# EUROPÄISCHE PATENTSCHRIFT

(11) **EP 2 080 806 B1**
(45) Veröffentlichungstag und Bekanntmachung des Hinweises auf die Patenterteilung: **09.04.2014**
(21) Anmeldenummer: 08169408.5
(22) Anmeldetag: 19.11.2008
(51) Int. Cl.: C12P 7/62, C12M 1/40

(54) **Verfahren zur heterogenkatalysierten Herstellung von Carbonsäurederivaten**
Method for heterogeneously catalysed manufacture of carboxylic acid derivatives
Procédé destiné à la fabrication hétérogène catalysée de dérivés d'acide carboxylique

(30) Priorität: 16.01.2008 DE 102008004725
(43) Veröffentlichungstag der Anmeldung: 22.07.2009
(73) Patentinhaber: Evonik Degussa GmbH, 45128 Essen (DE)
(72) Erfinder: Thum, Oliver Dr., 40880 Ratingen (DE); Hilterhaus, Lutz Dr., 21079 Hamburg (DE); Liese, Andreas Prof. Dr., 21077 Hamburg (DE)

(56) Entgegenhaltungen:
- EP-A- 0 413 307
- EP-A- 0 670 372
- DE-A1- 2 553 649
- DE-A1- 10 122 551
- US-A- 4 921 799
- US-A- 5 713 965

## Beschreibung

Die Erfindung betrifft ein neuartiges Verfahren zur Herstellung von Carbonsäurederivaten unter Verwendung von heterogenen Katalysatoren.

Carbonsäurederivate, wie beispielsweise Carbonsäureester oder -amide, stellen eine bedeutende Gruppe chemischer Verbindungen dar. Ester von Fettsäuren werden zum Beispiel häufig als Ölphase in kosmetischen Formulierungen eingesetzt. Zur Herstellung solcher Verbindungen ausgehend von Carbonsäuren oder aktivierten Derivaten, wie z. B. Säurechloriden, Anhydriden oder Estern kurzkettiger Alkohole, sind vielfältige Methoden bekannt. Eine erste Übersicht hierzu findet sich bei: Becker u.a., "Organikum", 22. Auflage, Wiley-VCH: 2004, Weinheim, Deutschland, Seite 472 ff.

Die Verwendung heterogener Katalysatoren hat den Vorteil, dass diese nach der Reaktion einfach abgetrennt und gegebenenfalls wiederverwendet werden können. Beispiele solcher heterogener Katalysatoren sind, neben im Reaktionsmedium unlöslichen festen Katalysatoren, z. B. Metallsalze, Ionenaustauscherharze oder auf geeigneten Trägern immobilisierte Katalysatoren. Besonders geeignet sind Katalysatoren mit Partikelgrößen, die ein einfaches und schnelles Abtrennen vom Reaktionsmedium erlauben. Häufig als Katalysator eingesetzt werden daher saure Ionenaustauscherharze, z. B. Amberlite IRA-118 (Fa. Rohm und Haas) und Enzyme oder Enzyme enthaltende Mikroorganismen, die auf Trägern immobilisiert sind. Als Träger für die Enzymimmobilisierung werden häufig Ionenaustauscherharze oder Polymerpartikel eingesetzt, die über geeignete Korngrößenverteilungen verfügen. Beispiele hierzu sind die Marktprodukte Novozym 435, Lipozym RM IM oder Lipozym TL IM der Firma Novozymes A/S, Bagsvaerd, Dänemark oder Amano PS, der Firma Amano, Japan. Aber auch viele andere Methoden zur Gewinnung immobilisierter Enzympräparate sind beschrieben, beispielsweise in K. Faber, "Biotransformations in Organic Chemistry", Springer: 2000, Berlin, Deutschland, 384ff., J. Am. Chem. Soc. 1999, 121, 9487-9496, J. Mol. Catal. B, 2005, 35, 93-99 oder die Patentanmeldung DE 10 2007 031689.7.

Eine weitere Bedingung zur Gewährleistung der Wiederverwendbarkeit ist die Minimierung der mechanischen Kräfte, die auf die eingesetzten Katalysatoren wirken und eine Desintegration des Trägers und somit eine deutliche Teilchengrößenreduzierung verursachen, wodurch die Anforderung der leichten Abtrennbarkeit nicht mehr erfüllt ist und oftmals auch große Aktivitätsverluste zu beobachten sind. Solche Kräfte treten vor allem auf, wenn konventionelle Rührreaktoren verwendet werden. Beispielsweise beschreibt DE 10 2007 031689.7 die auftretende Korngrößenreduktion von Enzymimmobilisaten nach Verwendung im Rührkolben. Da durch das erfindungsgemäße Verfahren im Reaktionsraum auf den Einsatz von Rührwerken verzichtet werden kann, und da die Durchmischung durch die Gaszufuhr gewährleistet ist, ist der mechanische Stress, aber auch die durch den Rührer induzierte thermische Belastung auf die Katalysatorteilchen auf ein Minimum reduziert. Dies sind Grundvoraussetzungen dafür, dass der Katalysator, bzw. das bevorzugt geträgerte enzymatische System wiederverwendet werden kann.

Eine Möglichkeit, die mechanische Stabilität der eingesetzten Katalysatoren zu verbessern liegt in dem Einsatz eines Festbettreaktors. Eur. J. Lipid Sci. Technol. 2003, 105, 601-607 beschreibt zum Beispiel den Einsatz eines Festbettreaktors zur Durchführung Lipase-katalysierter Veresterungen. Hier wird der Katalysator in Form eines gepackten Betts eingesetzt und die Reaktionsmischung aus einem Vorratsbehälter im Kreislauf durch das Festbett gepumpt, bis der gewünschte Umsatzgrad erreicht ist. Ein zusätzlicher Filtrationsschritt zur Abtrennung des Biokatalysators ist nicht nötig, da dieser im Festbett permanent zurück gehalten wird.

Durch die durch die Verwendung des Festbetts erhaltene Kompartimentierung des Systems, also die räumliche Trennung des relativ kleinen Katalysatorbehälters von dem relativ großen Vorratsbehälter, ergibt sich ein weiterer Vorteil: Für die gegebenenfalls erforderliche Reinigung des Systems oder den Austausch des Katalysators muss nur das Festbett geöffnet werden, welches systembedingt deutlich kleiner und leichter zugänglich ist als ein Rührbehälter, welcher über das gleiche Volumen verfügen müsste, wie das verwendete Vorratsgefäß. Gerade im Produktionsmaßstab, also bei Ansatzgröße im Bereich mehrerer Tonnen, ist eine Reinigung solcher Reaktorgefäße von Katalysatorpartikeln äußerst mühsam und umständlich.

Nachteilig an diesem Verfahren ist allerdings die Beschränkung auf niedrigviskose homogene Reaktionsmischungen, da hochviskose Mischungen oder Suspensionen aufgrund des hoher Druckverlustes nicht durch ein Festbett gefördert werden können. Die in Eur. J. Lipid Sci. Technol. 2003, 105, 601-607 oder Tens. Surfact. Det. 2004, 41, 287-290 beschriebenen Ester verfügen beispielsweise bei Reaktionstemperatur über Viskositäten von unter 10 mPas. Der Versuch, höherviskose Rohstoffe, wie zum Beispiel Polylycerin-3, welches bei Reaktionstemperatur über eine Viskosität von etwa 2000 mPas verfügt, in solch einem Reaktor zu prozessieren führt durch den deutlich gesteigerten Druckverlust zu so drastisch reduzierten erreichbaren Förderraten, dass eine ökonomisch sinnvolle Prozessführung nicht möglich ist. Die alternativ mögliche Verwendung von Lösungsmitteln ist üblicherweise unerwünscht, da dies zum einen zusätzliche Kosten verursacht (niedrigere Reaktorbeladung, Recyclingkosten, etc.) und zum anderen für viele Produktanwendung, z. B. in der kosmetischen oder pharmazeutischen Industrie die Abwesenheit von Lösungsmittelresten gewährleistet werden muss, oftmals bis in den einstelligen ppm Bereich. Letzteres ist zwar meist prinzipiell möglich, erfordert aber üblicherweise zusätzliche Aufarbeitungsschritte, wie z. B. Destillation, Extraktion oder Dämpfen, die zusätzliche Zeit in Anspruch nehmen, und daher die Herstellkosten weiter erhöhen und die Raum-Zeit-Ausbeuten der Verfahren senken.

Ein weiterer Nachteil des Festbettreaktors liegt in der teils inhomogenen Durchströmung des Reaktors unter Kanalbildung und der Möglichkeit des Verschließens der Hohlräume durch Schwebstoffe.

Es besteht daher weiterhin Bedarf an Reaktionsverfahren zur heterogenkatalysierten Herstellung von Carbonsäurederivaten, besonders aus hochviskosen Mischungen, welche die Nachteile des Standes der Technik überwinden.

Aufgabe der vorliegenden Erfindung war daher die Bereitstellung eines universellen Verfahrens zur heterogenkatalysierten Herstellung von Carbonsäurederivaten, welches die lösungsmittelfreie Prozessierung von niedrig- und höherviskosen Reaktionsmischungen bei gleichzeitiger Möglichkeit zur einfachen Abtrennung und Wiederverwendbarkeit des Katalysators ermöglicht. Vorzugsweise sollte das Verfahren zur Verwendung immobilisierter Biokatalysatoren geeignet sein.

Weitere nicht explizit genannte Aufgaben ergeben sich aus dem Kontext der nachfolgenden Beschreibung, der Beispiele sowie der Ansprüche.

Überraschender Weise wurde gefunden, dass diese Aufgabe durch ein Reaktorkonzept erfüllt wird, bei dem ein Umlaufreaktor mit einem Reaktionsbehälter eingesetzt wird, in dem der heterogene Katalysator in der Reaktionsmischung frei verteilt ist und ein zusätzlicher Gaseintrag für die Durchmischung im Reaktionsbehälter sorgt.

Gegenstand der vorliegenden Erfindung ist daher ein Verfahren zur Herstellung von Carbonsäurederivaten, welches dieses Reaktorkonzept nutzt.

Das erfindungsgemäße Verfahren wird nachfolgend beispielhaft beschrieben, ohne dass die Erfindung auf diese beispielhaften Ausführungsformen beschränkt sein soll. Sind nachfolgend Bereiche, allgemeine Formeln oder Verbindungsklassen angegeben, so sollen diese nicht nur die entsprechenden Bereiche oder Gruppen von Verbindungen umfassen, die explizit erwähnt sind, sondern auch alle Teilbereiche und Teilgruppen von Verbindungen, die durch Herausnahme von einzelnen Werten (Bereichen) oder Verbindungen erhalten werden können. Werden im Rahmen der vorliegenden Beschreibung Dokumente zitiert, so soll deren Inhalt vollständig zum Offenbarungsgehalt der vorliegenden Erfindung gehören. Werden im Rahmen der vorliegenden Erfindung Verbindungen, wie z. B. Polyether oder Polysiloxane, beschrieben, die verschiedene Einheiten mehrfach aufweisen können, so können diese statistisch verteilt (statistisches Oligomer) oder geordnet (Blockoligomer) in diesen Verbindungen vorkommen. Angaben zu Anzahl von Einheiten in solchen Verbindungen sind als Mittelwert, gemittelt über alle entsprechenden Verbindungen, zu verstehen.

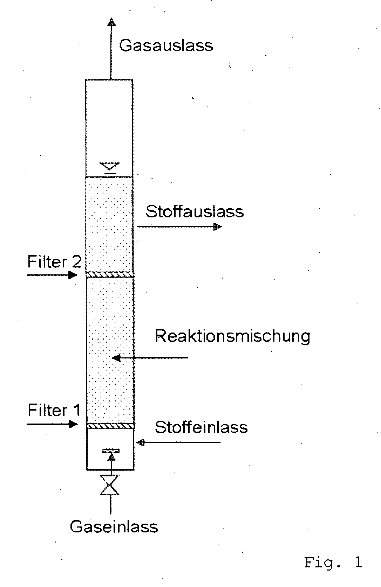

In dem erfindungsgemäßen Verfahren wird der eingesetzte Katalysator in einem Reaktionsgefäß R verwendet, welches dadurch gekennzeichnet ist, dass mindestens zwei Auslässe für das Reaktionsmedium, mindestens ein Gasauslass und mindestens eine Möglichkeit zur Gaseinleitung vorhanden sind. Darüber hinaus ist ein Teil des Reaktionsgefäßes durch geeignete Filter so abgetrennt, dass hierin der Katalysator eingesetzt werden kann. Dabei soll der Katalysator nicht dicht gepackt werden und die Ausführung des Reaktionsbehälters muss gewährleisten, dass sowohl Gasstrom als auch Stoffstrom durch den Bereich, bzw. die Bereiche führen, welcher, bzw. welche, den Katalysator enthalten. Eine bevorzugte Ausführungsform des Reaktionsgefäßes R ist in Fig. 1 dargestellt.

Die Gaseinleitung dient dazu, die Reaktionsmischung gleichmäßig zu durchmischen, den heterogenen Träger in Schwebe zu halten und, in Abhängigkeit der durchgeführten Reaktion, niedrigsiedende Reaktionsprodukte, beispielsweise Wasser bei Veresterungen oder niedere Alkohole bei Umesterungsreaktionen der entsprechenden Ester, aus der Reaktionsmischung zu entfernen und somit das Gleichgewicht zur Produktseite hin zu verschieben. Erfindungsgemäß bevorzugt wird der Gasauslass des Reaktionsgefäßes mit einem Kondensator verbunden, um die niedrigsiedenden Reaktionsprodukte abfangen zu können. Beispiele für erfindungsgemäße Gaseinleitungen stellen Ringbrausen, Gasdüsen, Keramikfilter, etc. dar, die dem Fachmann allgemein geläufig sind.

Erfindungsgemäß werden als Gase solche verwendet die mit den Reaktanden, dem Katalysator oder den Reaktormaterialien keine Reaktionen eingehen. Bevorzugt eingesetzt werden Luft, Magerluft, Sauerstoff, Stickstoff, Edelgase oder Kohlendioxid. Die eingesetzten Gase können aus geeigneten Druckbehältern, z. B. Gasflaschen, oder durch Kompressoren zugeführt werden. Der Abluftstrom kann abgeleitet oder durch geeignete Maßnahmen wiederverwendet und im Kreislauf geführt werden.

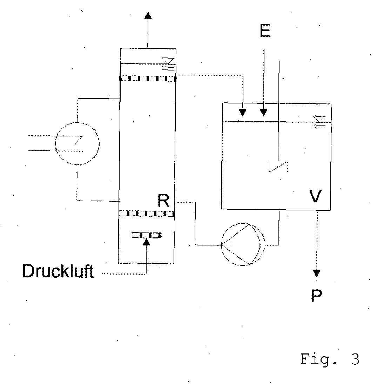

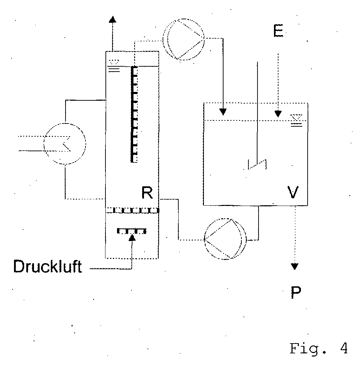

Erfindungsgemäß sind weitere Ausführungsformen des Reaktionsgefäßes R möglich. Beispielsweise ist es möglich, den Filter 1 in den Stoffeinlass zu integrieren oder als Kerzenfilter auszuführen. Beispiele für solche Ausführungsformen sind in Fig. 3 und Fig. 4 dargestellt. Alternativ lässt sich der Filter auch als Röhrbündel oder monolithischer Rohrbündel integrieren. Alternative Ausführungsformen des Reaktionsgefäßes R können auch ein Airlift-Reaktor, Druckschlaufenreaktor bzw. Mammut-Schlaufen Reaktor sein.

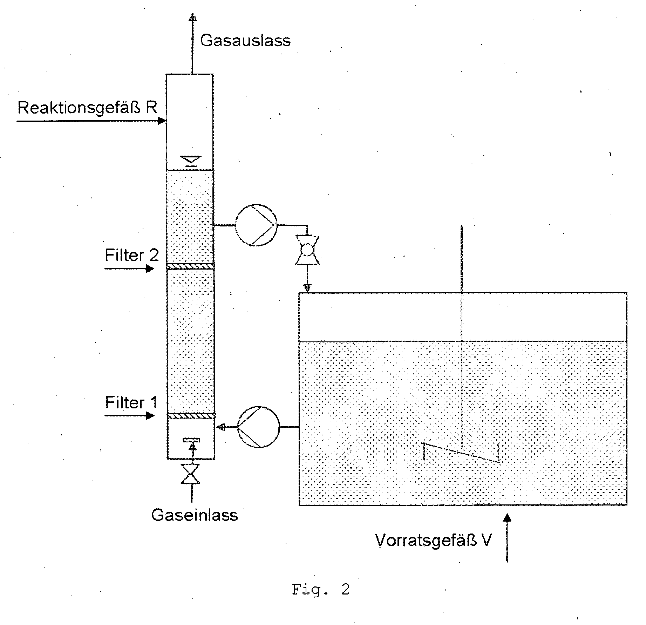

Erfindungsgemäß wird neben dem Reaktionsgefäß R mindestens ein Vorratsgefäß V verwendet. In einer Ausführungsform der Erfindung wird ein Vorratsgefäß verwendet, das mit mindestens 2 Stoffauslässen ausgestattet ist. Dabei wird die Reaktionsmischung aus dem Vorratsgefäß V über eine geeignete Leitung in das Reaktionsgefäß R gepumpt und nach Durchströmen des den Katalysator enthaltenden Bereichs wieder durch eine geeignete Leitung in das Vorratsgefäß zurückgepumpt. Dafür ist mindestens eine geeignete Pumpe zwischen Vorratsgefäß V und Reaktionsgefäß R erforderlich. Optional kann eine weitere Pumpe zwischen Reaktionsgefäß R und Vorratsgefäß V betrieben werden, wobei in diesem Fall die Pumpleistung der einzelnen Pumpen durch entsprechende Steuerungselemente aufeinander abzustimmen ist. Eine bevorzugte Ausführungsform der Gesamtanlage, bestehend aus Reaktionsgefäß R, Vorratsgefäß V, Leitungen und Pumpen ist in Fig. 2 gezeigt.

Das Vorratsgefäß V kann optional mit einem Rührwerk ausgestattet sein, um die Durchmischung des Reaktionssystems zu verbessern. Aus dem gleichen Grund kann die Zuleitung vom Vorratsgefäß V zum Reaktionsgefäß R mit einer weiteren technischen Vorrichtung zur Verbesserung der Durchmischung, beispielsweise einem Dispergator, unterbrochen sein.

In einer weiteren Ausführungsform der Erfindung ist das Vorratsgefäß V mit der Möglichkeit versehen, Vakuum anzulegen, um die Entfernung der niedrigsiedenden Reaktionsprodukte, beispielsweise Wasser bei Veresterungen oder niedere Alkohole bei Umesterungsreaktionen der entsprechenden Ester, aus der Reaktionsmischung weiter zu unterstützen. Bei der Verwendung von Vakuum kann es nötig sein, die Leitung zwischen Reaktionsgefäß R und Vorratsgefäß V durch eine zweite Pumpe sowie ein Druckhalteventil zu unterbrechen. Optional kann die Entfernung niedrigsiedender Reaktionskomponenten durch weitere Einbauten, beispielsweise Fallfilmverdampfer, weiter beschleunigt werden.

Die Bestandteile der erfindungsgemäßen Anlage, insbesondere das Reaktionsgefäß R, das Vorratsgefäß V und die Zu- und Ableitungen können mit einer Möglichkeit der Thermostatisierung versehen sein, beispielsweise mit einer Heizung auf Basis von Elektrizität, Dampf oder Thermalöl.

In einer weiteren besonderen Ausführungsform werden neben dem Reaktionsgefäß R zwei Vorratsgefäße V1 und V2 in einem kontinuierlichen Herstellungsverfahren verwendet. Dabei wird die Reaktionsmischung aus dem, mit mindestes einem Stoffauslass und ggf. mit einem Rührwerk oder einer anderen Mischereinrichtung versehenen, Vorratsgefäß V1 wie oben beschrieben in das Reaktionsgefäß R gefördert. Nach Durchströmen des den Katalysator enthaltenen Bereichs wird die Reaktionsmischung in den Vorratsbehälter V2 befördert.

In einer weiteren Ausführungsform kann die Reaktionsmischung aus dem Vorratsbehälter V2 durch ein zweites Reaktionsgefäß R2 in einen Vorratsbehälter V3 gefördert werden, wobei das Reaktionsgefäß R2 wie für den Behälter R1 beschrieben aufgebaut und mit einem Katalysator gefüllt ist und der Vorratsbehälter V2 wie der Behälter V1 mit mindestes einem Stoffauslass und ggf. mit einem Rührwerk oder einer anderen Mischereinrichtung versehenen ist.

Als Katalysatoren können erfindungsgemäß solche verwendet werden, deren Korngröße so beschaffen ist, dass sie mit den üblichen verfügbaren Filtersystemen ohne großen Druckverlust in dem Reaktionsbehälter zurückgehalten werden können, also größer 0,5 µm, bevorzugt größer 5 µm, besonders bevorzugt größer 10 µm, insbesondere größer 25 µm.

Dabei kann es sich um polymere Katalysatoren entsprechender Korngröße oder um auf geeigneten Trägern immobilisierte Katalysatoren handeln.

Beispiele für die polymeren Katalysatoren sind Ionenaustauscher, wie z. B. sulfonierte Polystyrene oder Zeolite.

Erfindungsgemäß können als auf geeigneten Trägern immobilisierte Katalysatoren chemische Katalysatoren oder Enzymimmobilisate eingesetzte werden.

Beispiele für chemische Katalysatoren sind trägergebundene Lewis Säuren und amphotere Hydroxide.

Zur Herstellung der Enzymimmobilisate können ganze Zellen, ruhende Zellen, gereinigte Enzyme oder Zellextrakte, die die entsprechenden Enzyme enthalten oder Mischungen davon eingesetzt werden. Bevorzugt eingesetzt werden hydrolytische Enzyme, z. B. Lipasen, Esterasen oder Proteasen, wie beispielsweise Lipasen aus *Candida rugosa, Candida antarctica, Pseudomonas sp., Thermomyces langosiosus,* Schweinepankreas, *Mucor miehei, Alcaligines sp.,* Cholesterolesterase aus *Candida rugosa,* Esterase aus der Schweineleber, besonders bevorzugt Lipasen, eingesetzt. Demgemäß weisen die Enzymimmobilisate vorzugsweise Enzyme aus der Klasse der Hydrolasen, bevorzugt Lipasen, auf.

Als Träger zur Immobilisierung der Enzyme oder Enzyme enthaltenden Mikroorganismen können inerte organische oder anorganische Träger verwendet werden. Vorzugsweise werden als inerte Träger solche partikulären Träger verwendet bzw. sind im Enzymimmobilisat vorhanden, die eine Teilchengrößenverteilung aufweisen, bei der mindestens 90% der Teilchen eine Teilchengröße von 0,5 bis 5000 µm, bevorzugt von 10 µm bis 2000 µm, besonders bevorzugt von 25 µm bis 2000 µm aufweisen. Als organische Träger können insbesondere solche eingesetzt werden, die Polyacrylat, Polymethacrylat, Polyvinylstyrol, Styrol-Divinylbenzolcopolymeren, Polypropylen, Polyethylen, Polyethylenperepthalat, PTFE und/oder andere Polymere aufweisen oder aus diesen bestehen. Als Trägermaterial können, in Abhängigkeit von dem zu immobilisierenden Enzym, insbesondere saure oder basische Ionenaustauscherharze eingesetzt werden, beispielsweise Duolite A568, Duolite XAD 761, Duolite XAD 1180, Duolite XAD 7HP, Amberlite IR 120, Amberlite IR 400, Amberlite CG 50, Amberlyst 15 (alles Produkte der Fa. Rohm und Haas) oder Lewatit CNP 105 und Lewatit VP OC 1600 (Produkte der Fa. Lanxess, Leverkusen, Deutschland). Als anorganische Träger können aus dem Stand der Technik bekannte, oxidische und/oder keramische Träger eingesetzt werden. Insbesondere können als anorganische Träger beispielsweise Celite, Zeoliten, Silica, Controlled-Pore Glas (CPG) oder andere Träger, wie sie zum Beispiel in L. Cao, "Carrier-bound Immobilized Enzymes: Principles, Application and Design", Wiley-VCH: 2005, Weinheim, Deutschland, beschrieben sind, eingesetzt werden. Besonders bevorzugt bestehen die im Enzymimmobilisat vorhandenen inerten Träger bzw. die zur Herstellung der Enzymimmobilisate verwendeten inerten Träger aus Polyvinylstyrol, Polymethacrylat oder Polyacrylat.

Die Immobilisierung auf den Partikeln kann erfindungsgemäß kovalent oder nichtkovalent erfolgen.

Beispiele für die erfindungsgemäß eingesetzten Enzymimmobilisate sind Novozym 435, Lipozym RM IM oder Lipozym TL IM der Firma Novozymes A/S, Bagsvaerd, Dänemark oder Amano PS, der Firma Amano, Japan.

Gegenstand der vorliegenden Erfindung ist somit ein Verfahren zur heterogenkatalysierten Herstellung von Carbonsäurederivaten unter Verwendung eines Reaktorensystems beispielsweise nach Figur 2 enthaltend mindestens einen Vorratsbehälter V, Zuleitungen, mindestens eine Pumpe und ein Reaktionsgefäß R, wobei durch das Reaktionsgefäß R durch eine Gaszuleitung ein permanenter Gasstrom angelegt ist und in welchem ein Reaktionsraum, in dem der heterogene Katalysator nicht dicht gepackt eingefüllt ist, so durch mindestens zwei Filter abgetrennt ist, dass sowohl der Stoff- als auch der Gasstrom durch den Reaktionsraum geleitet werden.

Ein weiterer Gegenstand ist ein Verfahren bei dem als Katalysator mindestens ein Enzym, oder mindestens ein Enzym enthaltende Mikroorganismen eingesetzt werden.
Ein weiterer Gegenstand ist ein Verfahren bei dem das enzymatische System heterogen geträgert auf einem Feststoff vorliegt.
Ein weiterer Gegenstand ist ein Verfahren bei dem der enzymatische Katalysator partikulär auf einem Trägermaterial kovalent oder nicht-kovalent fixiert ist.
Ein weiterer Gegenstand ist ein Verfahren bei dem der Feststoff aus einem Polymer auf Basis von Polyacrylat, Polymethacrylat, Polyvinylstyrol, Styrol-Divinylbenzolcopolymeren, Polypropylen, Polyethylen, Polyethylenperepthalat, oder Polytetrafluorethylen oder Copolymeren dieser Verbindungen oder oxidischen und/oder keramischen Trägern besteht.
Ein weiterer Gegenstand ist ein Verfahren bei dem der Feststoff aus einem sauren oder basischen Ionenaustauscherharz besteht.
Ein weiterer Gegenstand ist ein Verfahren bei dem der oxidischen und/oder keramischen Träger aus Celite, Zeolite, Silica oder Controlled-Pore Glas besteht.
Ein weiterer Gegenstand ist ein Verfahren bei dem das Trägermaterial eine mittlere Korngröße von größer als 0,5 µm aufweist.
Ein weiterer Gegenstand ist ein Verfahren bei dem das heterogene Katalysatorsystem wiederverwendet wird.
Ein weiterer Gegenstand ist ein Verfahren bei dem am Vorratsbehälter V Vakuum angelegt wird.
Ein weiterer Gegenstand ist ein Verfahren bei dem als Gas Luft, Magerluft, Sauerstoff, Stickstoff, Edelgase oder Kohlendioxid, bevorzugt Luft oder Stickstoff, eingesetzt werden.

Ein weiterer Gegenstand ist ein Verfahren bei dem die Reaktion bei einer Temperatur von 20 °C bis 100 °C durchgeführt wird.

Weitere Gegenstände der Erfindung ergeben sich aus dem Wortlaut der Ansprüche.

Erfindungsgemäß wird das Verfahren bevorzugt eingesetzt zur Durchführung von Umsetzungen der allgemeinen Formel I 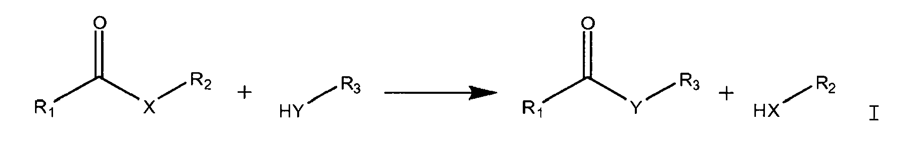 wobei
- R₁: der Acylrest linearer, verzweigter, gesättigter oder ungesättigter, gegebenenfalls zusätzlich substituierter Carbonsäuren mit 2 bis 30 C-Atomen,
- X: gleich Sauerstoff, Schwefel oder -NH-, bevorzugt Sauerstoff
- R₂: Wasserstoff oder eine lineare oder verzweigte Alkyl- oder Alkenylgruppe mit 1 bis 3 Kohlenstoffatomen,
- Y: gleich Sauerstoff, Schwefel oder -NH-, bevorzugt Sauerstoff oder -NH-, besonders bevorzugt Sauerstoff,
- R₃: ein linearer, verzweigter, gesättigter oder ungesättigter, gegebenenfalls zusätzlich substituierter Alkylrest mit 2 bis 30 C-Atomen ist.

Erfindungsgemäß können die optionalen zusätzlichen Substituenten der Reste R₁ und R₃ unabhängig voneinander beispielsweise Hydroxygruppen, Estergruppen, Polyestergruppen, Carbonatgruppen, Polycarbonatgruppen, Ethergruppen, Polyethergruppen, Urethangruppen, Polyurethangruppen, Amidgruppen, Polyamidgruppen, Alkylamingruppen, Dialkylamingruppen, Halogenide, Siloxangruppen, Estergruppen anorganischer Säuren, Sulfate, Phosphate oder ähnliche Gruppierungen sein.

In einer bevorzugten Ausführungsform stellt R₁ den Acylrest handelsüblichen Säuren, wie beispielsweise Essigsäure, Propansäure, Butansäure, Pentansäure, Chloressigsäure, Trifluoressigsäure, Ethylhexansäure, Isononansäure, Isotridecansäure oder Isostearinsäure dar.

In einer weiteren bevorzugten Ausführungsform werden als R₁ Acylreste natürliche Fettsäuren auf Basis natürlicher pflanzlicher oder tierischer Öle eingesetzt. Bevorzugt werden natürliche Fettsäuren wie z.B. Capronsäure, Caprylsäure, Caprinsäure, Laurinsäure, Myristinsäure, Palmitinsäure, Palmitoleinsäure, Isostearinsäure, Stearinsäure, 12-Hydroxystearinsäure, Dihydroxystearinsäure, Ölsäure, Linolsäure, Petroselinsäure, Elaidinsäure, Arachinsäure, Behensäure, Erucasäure, Gadoleinsäure, Linolensäure, Eicosapentaensäure, Docosahexaensäure oder Arachidonsäure, allein oder in Mischung eingesetzt. Der Rest R₁ kann ebenfalls der Acylrest von Polykondensationsprodukten von hydroxyfunktionalisierten Säuren, beispielsweise Poly-12-hydroxystearinsäure oder Polyricinolsäure sein. Bei dem als Rest R₁ eingesetzten Acylresten kann es sich um technische Mischungen handeln, beispielsweise um Mischungen von natürlichen Fettsäuren, z. B. Rapsölfettsäure, Sojaölfettsäure, Sonnenblumenölfettsäure, Talgölfettsäure, Palmölfettsäure, Palmkernölfettsäure, Kokosfettsäure, die in Abhängigkeit von ihrer konkreten Quelle und den eingesetzten Aufreinigungsverfahren in ihrer genauen Zusammensetzung Schwankungen unterworfen sein können, sowie typische Nebenbestandteile, wie ungesättigte, funktionalisierte oder verzweigte Komponenten enthalten können. Darüber hinaus können auch Mischungen von Säuren anderer Herkunft, zum Beispiel auf Basis petrochemischer Verfahren, eingesetzt werden.

In einer weiteren bevorzugten Ausführungsform werden als R₃ beispielsweise die Kohlenwasserstoffreste von Propanol, Butanol, Pentanol, Hexanol, Octanol oder deren Isomeren wie Isopropanol, Isobutanol, 2-Ethylhexanol, Isononylalkohol, Isotridecylalkohol, mehrwertige Alkohole, wie 1,6-Hexandiol, 1,2-Pentandiol, Dihydroxyaceton, 1,2-Propylenglykol, 1,3-Propylenglykol, Neopentylglykol, Trimethylolpropan, Pentaerithrol, Sorbitol, Glycerin, Diglycerin, Triglycerin, Polyglycerin, Ethylenglykol, Diethylenglykol, Triethylenglykol, Polyethylenglykol, oder aminofunktionalisierte Alkohole, wie N,N-Dimethylethanolamin, eingesetzt. Weitere Beispiele sind die Kohlenwasserstoffreste von Alkoholen, die nach bekannten Verfahren aus einbasischen Fettsäuren auf Basis natürlicher pflanzlicher oder tierischer Öle mit 6 bis 30 Kohlenstoffatomen, insbesondere mit 8 bis 22 Kohlenstoffatomen, hergestellt werden, beispielsweise Capronsäure, Caprylsäure, Caprinsäure, Laurinsäure, Myristinsäure, Palmitinsäure, Palmitoleinsäure, Isostearinsäure, Stearinsäure, 12-Hydroxystearinsäure, Dihydroxystearinsäure, Ölsäure, Linolsäure, Petroselinsäure, Elaidinsäure, Arachinsäure, Behensäure, Erucasäure, Gadoleinsäure, Linolensäure, Eicosapentaensäure, Docosahexaensäure oder Arachidonsäure, allein oder in Mischung eingesetzt.

Beispiele für die erfindungsgemäßen polyethersubstituierten Reste R₃ sind die Alkylreste von Polyethylenglykol, Polypropylenglykol, Polybutylenglykol, Polystryroloxid oder Copolymere von mindestens zwei Monomeren aus der Gruppe Ethylenglykol, Propylenglykol, Butylenglykol Stryroloxid, sowie Polyglycerin. Diese polyethersubstituierten Reste können ihrerseits wieder Substituenten an weiteren OH-Gruppen tragen, beispielsweise Alkyl- oder Alkenylether oder Alkylester.

Beispiele für die erfindungsgemäßen polyestersubstituierten Reste R₃ sind freie OH-Gruppen tragende Polymere auf Basis von ε-Caprolacton oder γ-Valerolacton, beispielsweise Placcel L212AL der Firma Daicel.

Beispiele für die erfindungsgemäßen polycarbonatsubstituierten Reste R₃ sind freie OH-Gruppen tragende Polymere auf Basis von Dialkylcarbonat- und Dihydroxyalkyleinheiten, beispielsweise Placcel CD220 der Firma Daicel.

Beispiele für die erfindungsgemäßen polysiloxansubstituierten Reste R₃ sind freie Alkyl-OH-Gruppen tragende organomodifizierte Polysiloxane, wie sie beispielsweise durch Hydrosilylierung von SiH-Siloxanen mit terminal ungesättigten Alkoholen nach dem Fachmann bekannten Methoden zugänglich sind. Insbesondere können hier lineare Alkenole, wie 5-Hexen-1-ol oder Allyloxyderivate, wie Allyloxyethanol, Allylglycerin, Allylpolyglycerin, Allyltrimethylolpropan, Allylpolyethylenglykol, Allylpolypropylenglykol, oder Allylpolyethylenglykol-polyproypylenglykol-copolymere, verwendet werden.

Beispiele für die erfindungsgemäßen polysiloxansubstituierten Reste R₁ sind freie Carboxygruppen tragende organomodifizierte Polysiloxane, wie sie beispielsweise durch Hydrosilylierung von SiH-Siloxanen mit terminal ungesättigten Säuren nach dem Fachmann bekannten Methoden zugänglich sind.

Insbesondere können hier Acrylsäure, Methacrylsäure oder Undec-10-ensäure verwendet werden.

Die nachfolgenden Beispiele sollen die vorliegende Erfindung näher erläutern, ohne den Schutzbereich einzuschränken, der sich aus der Beschreibung und den Patentansprüchen ergibt.

### Materialien und Methoden:

Novozym 435 (NZ435) ist ein kommerzielles Enzymimmobilisat der Firma Novozymes A/S, Bagsvaerd/Dänemark, einer auf einem Polymethyacrylat durch Adsorption immobilisierten Lipase B aus C. *antarctica.*

### Beispiele

### Beispiel 1: Aufbau Versuchreaktor:

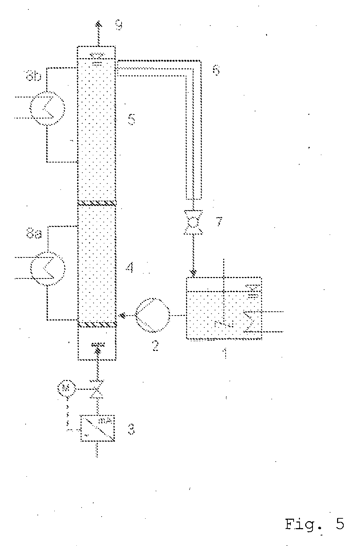

Der Aufbau des beispielhaften Versuchreaktors ist schematisch in Fig. 5 dargestellt. Der Umlaufreaktor besteht aus einem thermostatisierten (8) Reaktionsgefäß (4, 5) mit einer Länge von 60 cm und einem Durchmesser von 2,5 cm. Das Reaktionsgefäß ist durch ein mittig angebrachtes, herausnehmbares Metallsieb mit einer Porenweite von 50 µm in zwei Bereiche getrennt (4, 5). Ein weiteres Metallsieb begrenzt das Reaktionsgefäß nach unten und dient als Gasverteiler. In dem unteren Bereich des Reaktionsgefäßes (4) wird somit der heterogene Katalysator zurückgehalten. Aus dem gerührten Vorlagebehälter (1) wird die Reaktionsmischung oberhalb des Gasverteilers in das Reaktionsgefäß (4) mit Hilfe einer Schlauchpumpe (2) gepumpt. Gleichzeitig wird von unten kontrolliert Gas in das Reaktionsgefäß eingetragen (3). Die Reaktionsmischung füllt zuerst den unteren Bereich (4), dann den oberen Bereich (5), wobei der Katalysator durch die Siebe zurückgehalten wird. Bei Erreichen des Überlaufs fließt die Reaktionsmischung über eine thermostatisierte Leitung (6, 7) zurück in den Vorlagebehälter (1). Das eingetragene Gas entweicht über den Gasauslass (9).
Der Austrag des Reaktionswassers, bzw. der anderen niedrigsiedenden Reaktionsprodukte, und die Durchmischung der Reaktanden erfolgt im Reaktionsgefäß durch die aufsteigenden Gasblasen. Gleichzeitig wird der Vorlagebehälter gerührt wobei zusätzlich das Anlegen von Vakuum am Vorlagebehälter zum beschleunigten Austrag der niedrigsiedenden Reaktionsprodukte möglich ist.

### Beispiel 2: Synthese von Myristyl Myristat

329 g Myristylalkohol und 351 g Myristinsäure wurden in einem 1L-Vorlagebehälter auf 60 °C temperiert und durch einen Magnetrührer durchmischt (Viskosität der Mischung bei 60° C ca. 6 mPas). In dem unteren Bereich des Reaktionsbehälters wurde 2.7 g Novozym 435 vorgelegt und der gesamte Reaktionsbehälter auf 60 °C temperiert. Der Drucklufteintrag wurde auf 0,4 L/min eingeregelt und anschließend die Schlauchpumpe auf ∼ 20 mL/min eingestellt und eingeschaltet. Der Rücklauf war auf 60 °C temperiert. Der Reaktionsverlauf wurde für ∼25 h verfolgt, wobei ein Umsatz von 99,3 % bezogen auf die Fettsäure erreicht wurde.

**Tabelle 1: Umsatzdaten zu Beispiel 2:**

| Zeit [min] | Umsatz [%] |
|---|---|
| 0 | 0 |
| 30 | 25,8% |
| 60 | 46,9% |
| 124 | 74,6% |
| 169 | 85,7% |
| 223 | 92,6% |
| 300 | 96,5% |
| 346 | 97,4% |
| 1442 | 99,3% |

### Beispiel 3: Wiederverwendung des Katalysators

Analog zu Beispiel 2 werden mehrere Reaktionen zur Synthese von Myristylmyristat unter Wiederverwendung des Enzymkatalysators ohne Zwischenreinigung durchgeführt und der Umsatz nach 1 und 24 Stunden bestimmt. Es wurden 10 Wiederholungen durchgeführt

**Tabelle 2: Umsatzdaten zu Beispiel 3**

| Wiederholung | Umsatz 1 h | Umsatz 24 h |
|---|---|---|
| 1 | 46,9 % | 99,3 % |
| 2 | 45,3 % | 99,6 % |
| 3 | 46,2 % | 99,3 % |
| 4 | 44,7 % | 99,5 % |
| 5 | 47,1 % | 99,1 % |
| 6 | 46,9 % | 99,2 % |
| 7 | 44,7 % | 99,0 % |
| 8 | 47,4 % | 98,9 % |
| 9 | 45,2 % | 99,2 % |
| 10 | 44,3 % | 99,0 % |

### Beispiel 4: Synthese von Polyglycerin-3 Laurat

350 g Polyglycerin-3 (Fa. Daicel) und 280 g Laurinsäure wurden in einem 1L-Vorlagebehälter auf 80 °C temperiert und durch einen mechanischen Rührer durchmischt (Viskosität der Mischung bei 60° C ca. 2000 mPas). In dem unteren Bereich des Reaktionsbehälters wurde 5,5 g Novozym 435 vorgelegt und der gesamte Reaktionsbehälter auf 80 °C temperiert. Der Drucklufteintrag wurde auf 1,20 L/min eingeregelt und anschließend die Schlauchpumpe auf ∼ 20 mL/min eingestellt und eingeschaltet. Der Rücklauf war auf 80 °C temperiert. Der Reaktionsverlauf wurde für ∼24 h verfolgt, wobei ein Umsatz von 96,7 % bezogen auf die Fettsäure erreicht wurde.

**Tabelle 3: Umsatzdaten zu Beispiel 4:**

| Zeit [min] | Umsatz [%] |
|---|---|
| 0 | 0,0 |
| 15 | 14,3 |
| 30 | 27,9 |
| 60 | 49,7 |
| 123 | 72,7 |
| 244 | 90,3 |
| 1440 | 96,7 |

## Patentansprüche

1. Verfahren zur heterogenkatalysierten Herstellung von Carbonsäurederivaten unter Verwendung eines Reaktorensystems enthaltend mindestens einen Vorratsbehälter V, Zuleitungen, mindestens eine Pumpe und ein Reaktionsgefäß R, **dadurch gekennzeichnet, dass** durch das Reaktionsgefäß R durch eine Gaszuleitung ein permanenter Gasstrom, dessen Gase mit den Reaktanden, dem Katalysator oder den Reaktormaterialien keine Reaktionen eingehen, angelegt ist und in welchem ein Reaktionsraum, in dem der heterogene Katalysator nicht dicht gepackt eingefüllt ist, so durch mindestens zwei Filter abgetrennt ist, dass sowohl der Stoff- als auch der Gasstrom durch den Reaktionsraum geleitet werden, wobei die Gase des Gasstroms die Reaktionsmischung gleichmäßig durchmischen.

2. Verfahren nach Anspruch 1, **dadurch gekennzeichnet, dass** als Katalysator mindestens ein Enzym, oder mindestens ein Enzym enthaltende Mikroorganismen eingesetzt werden.

3. Verfahren nach Anspruch 2, **dadurch gekennzeichnet, dass** das enzymatische System heterogen geträgert auf einem Feststoff vorliegt.

4. Verfahren nach Anspruch 3, **dadurch gekennzeichnet, dass** der enzymatische Katalysator partikulär auf einem Trägermaterial kovalent oder nicht-kovalent fixiert ist.

5. Verfahren nach Anspruch 3 oder 4, **dadurch gekennzeichnet, dass** der Feststoff aus einem Polymer auf Basis von Polyacrylat, Polymethacrylat, Polyvinylstyrol, Styrol-Divinylbenzolcopolymeren, Polypropylen, Polyethylen, Polyethylenperepthalat, oder Polytetrafluorethylen oder Copolymeren dieser Verbindungen oder oxidischen und/oder keramischen Trägern besteht.

6. Verfahren nach Anspruch 3 oder 4, **dadurch gekennzeichnet, dass** der Feststoff aus einem sauren oder basischen Ionenaustauscherharz besteht.

7. Verfahren nach Anspruch 3 oder 4, **dadurch gekennzeichnet, dass** der oxidischen und/oder keramischen Träger aus Celite, Zeolite, Silica oder Controlled-Pore Glas besteht.

8. Verfahren nach einem der Ansprüche 3 bis 7, **dadurch gekennzeichnet, dass** das Trägermaterial eine mittlere Korngröße von größer als 0,5 µm aufweist.

9. Verfahren nach einem der Ansprüche 1 bis 8, **dadurch gekennzeichnet, dass** das heterogene Katalysatorsystem wiederverwendet wird.

10. Verfahren nach einem der Ansprüche 1 bis 9, **dadurch gekennzeichnet, dass** am Vorratsbehälter V Vakuum angelegt wird.

11. Verfahren nach einem der Ansprüche 1 bis 10, **dadurch gekennzeichnet, dass** als Gas Luft, Magerluft, Sauerstoff, Stickstoff, Edelgase oder Kohlendioxid, bevorzugt Luft oder Stickstoff, eingesetzt werden.

12. Verfahren nach einem der Ansprüche 1 bis 11, **dadurch gekennzeichnet, dass** die Reaktion bei einer Temperatur von 20 °C bis 100 °C durchgeführt wird.

## Claims

1. Process for heterogeneously catalysed preparation of carboxylic acid derivatives using a reactor system comprising at least one reservoir vessel V, feed lines, at least one pump and a reaction vessel R, **characterized in that** a permanent gas flow, the gases in which do not enter into any reactions with the reactants, the catalyst or the reactor materials, through the reaction vessel R is applied through a gas feed line, and in which a reaction chamber in which the heterogeneous catalyst is not introduced in tightly packed form is delimited by at least two filters such that both the mass flow and the gas flow are passed through the reaction chamber with homogenous mixing of the reaction mixture by the gases in the gas flow.

2. Process according to Claim 1, **characterized in that** the catalyst used is at least one enzyme, or microorganisms comprising at least one enzyme.

3. Process according to Claim 2, **characterized in that** the enzymatic system is present in heterogeneously supported form on a solid.

4. Process according to Claim 3, **characterized in that** the enzymatic catalyst is fixed covalently or noncovalently in particulate form on a support material.

5. Process according to Claim 3 or 4, **characterized in that** the solid consists of a polymer based on polyacrylate, polymethacrylate, polyvinylstyrene, styrene-divinylbenzene copolymers, polypropylene, polyethylene, polyethylene terephthalate, or polytetrafluoroethylene or copolymers of these compounds or oxidic and/or ceramic supports.

6. Process according to Claim 3 or 4, **characterized in that** the solid consists of an acidic or basic ion exchange resin.

7. Process according to Claim 3 or 4, **characterized in that** the oxidic and/or ceramic support consists of Celite, zeolites, silica or controlled-pore glass.

8. Process according to any one of Claims 3 to 7, **characterized in that** the support material has a mean particle size of greater than 0.5 µm.

9. Process according to any one of Claims 1 to 8, **characterized in that** the heterogeneous catalyst system is reused.

10. Process according to any one of Claims 1 to 9, **characterized in that** vacuum is applied at the reservoir vessel V.

11. Process according to any one of Claims 1 to 10, **characterized in that** the gas used is air, lean air, oxygen, nitrogen, noble gases or carbon dioxide, preferably air or nitrogen.

12. Process according to any one of Claims 1 to 11, **characterized in that** the reaction is performed at a temperature of 20°C to 100°C.

## Revendications

1. Procédé pour la préparation catalysée par voie hétérogène de dérivés d'acides carboxyliques en utilisant un système de réacteurs contenant au moins un réservoir V, des conduites d'alimentation, au moins une pompe et un récipient de réaction R, **caractérisé en ce qu'**un flux gazeux permanent, dont les gaz n'entrent pas en réaction avec les réactifs, le catalyseur ou les matériaux du réacteur, est instauré au travers du récipient de réaction R via une conduite d'alimentation de gaz et dans lequel un espace de réaction, dans lequel le catalyseur hétérogène est introduit de manière non compacte, est séparé par au moins deux filtres de manière telle que tant le flux de matières que le flux de gaz sont guidés au travers de l'espace de réaction, les gaz du flux gazeux mélangeant de manière régulière le mélange réactionnel.

2. Procédé selon la revendication 1, **caractérisé en ce qu'**on utilise comme catalyseur au moins une enzyme ou des microorganismes contenant au moins une enzyme.

3. Procédé selon la revendication 2, **caractérisé en ce que** le système enzymatique se trouve sous forme supportée, de manière hétérogène, sur un solide.

4. Procédé selon la revendication 3, **caractérisé en ce que** le catalyseur enzymatique est fixé de manière covalente ou non covalente sous forme particulaire sur un matériau support.

5. Procédé selon la revendication 3 ou 4, **caractérisé en ce que** le solide est constitué par un polymère à base de polyacrylate, de polyméthacrylate, de polyvinylstyrène, de copolymères de styrène-divinylbenzène, de polypropylène, de polyéthylène, de poly(téréphtalate d'éthylène) ou de polytétrafluoroéthylène ou par des copolymères de ces composés ou par des supports oxydes et/ou céramiques.

6. Procédé selon la revendication 3 ou 4, **caractérisé en ce que** le solide est constitué par une résine échangeuse d'ions acide ou basique.

7. Procédé selon la revendication 3 ou 4, **caractérisé en ce que** le support oxyde et/ou céramique est constitué par de la célite, une zéolite, de la silice ou du verre à pores contrôlés.

8. Procédé selon l'une quelconque des revendications 3 à 7, **caractérisé en ce que** le matériau support présente une grosseur moyenne des grains supérieure à 0,5 µm.

9. Procédé selon l'une quelconque des revendications 1 à 8, **caractérisé en ce que** le système catalytique hétérogène est réutilisé.

10. Procédé selon l'une quelconque des revendications 1 à 9, **caractérisé en ce qu'**un vide est instauré dans le réservoir V.

11. Procédé selon l'une quelconque des revendications 1 à 10, **caractérisé en ce qu'**on utilise, comme gaz, de l'air, de l'air maigre, de l'oxygène, de l'azote, des gaz nobles ou du dioxyde de carbone, de préférence de l'air ou de l'azote.

12. Procédé selon l'une quelconque des revendications 1 à 11, **caractérisé en ce que** la réaction est réalisée à une température de 20 à 100°C.
